# EUROPEAN PATENT APPLICATION

(11) **EP 3 269 373 A1**
(43) Date of publication of application: **17.01.2018**
(21) Application number: 16178913.6
(22) Date of filing: 11.07.2016
(51) Int. Cl.: A61K 31/58, A61K 9/00, A61P 5/28, A61P 5/32

(54) **LOW-DOSED ORAL DOSAGE FORM OF AN AKR1C3 INHIBITOR FOR TREATMENT OF DISEASES**

(71) Applicant: Bayer Pharma Aktiengesellschaft, 13353 Berlin (DE)
(72) Inventor: Ingwersen, Jan-Peter, 13465 Berlin (DE); Gröttrup-Wolfers, Esther, 14129 Berlin (DE); Hilpert, Jan, 10115 Berlin (DE); Müller, Simone, 13465 Berlin (DE)
(74) Representative: BIP Patents

(57) **Abstract**

The present invention relates to a low-dosed oral dosage form for treatment and/or prophylaxis of diseases, especially of endometriosis, polycystic ovary syndrome and atopic dermatitis. More particularly, the present invention concerns oral dosage forms comprising up to 120 mg N-{[17-(5-Fluoropyridin-3-yl)estra-1,3,5(10),16-tetraen-3-yl]carbonyl}-N-methyl-β-alanine per day..

## Description

### FIELD OF THE INVENTION

The present invention relates to a low-dosed oral dosage form for treatment and/or prophylaxis of diseases, especially of endometriosis, polycystic ovary syndrome and atopic dermatitis. More particularly, the present invention concerns oral dosage forms comprising up to120 mg *N*-{[17-(5-Fluoropyridin-3-yl)estra-1,3,5(10),16-tetraen-3-yl]carbonyl}-*N*-methyl-*β*-alanine / 70 kg body weight per day for women.

### BACKGROUND OF THE INVENTION

Endometriosis is a chronic, mainly estrogen-dependent disease characterized by the presence of endometrial tissue outside the uterine cavity. Endometriosis affects 10-20% of women in their reproductive age and has a devastating impact on their quality of life. Major symptoms of endometriosis are chronic pelvic pain and subfertility.

Suspected endometriosis is treated initially with nonsteroidal anti-inflammatory drugs, combined oral contraceptives (COCs) (which are both used off label) or progesterone receptor agonists (dienogest, Visanne). After failure of this initial drug treatment, the current gold standard treatment option is the surgical ablation of the endometriotic lesions in a laparoscopic procedure. This procedure is accompanied with a high recurrence rate. Currently, there is no long-term medication available in patients in which COCs and progestins failed. The treatment with Gonadotropin Releasing Hormone (GnRH) agonists as second-line therapy is only approved for short-term treatment (6 months), since GnRH analogues lead to a chemical castration, including menopausal side-effects like bone-mass-loss and hot flushes. New and long-term treatment options with reduced side effects and high efficacy for treatment refractory endometriosis patients are urgently needed.

Estrogen (E2) deprivation is the clinically proven concept and the underlying primary mechanism of action for pharmacological treatment of endometriosis. Besides systemic estrogen levels, there is increasing evidence that locally derived estrogen contributes to the growth of endometriotic lesions. High intra-tissue estrogen concentrations in endometriotic lesions have recently been described, suggesting high local estrogen synthesis in endometriosis (Huhtinen et al. (2012), J Clin Endocrinol Metab.; 97(11):4228-4235). Accordingly, inhibition of local E2 production in the endometriotic lesion is regarded as a highly attractive mechanism of action for the treatment of endometriosis. Compounds addressing the inhibition of local E2 synthesis are currently in development (aromatase inhibitor + progestin).

In addition, a role of progesterone in endometriosis has also been described. Systemic administration of progesterone receptor agonists (dienogest, Visanne) has proven to be efficacious in endometriosis. Aside from hormonal drivers, prostaglandins have been described to play a role in the pathogenesis of endometriosis, in particular prostaglandin F2 alpha as a driver of inflammation (Lousse et al. (2012). Front Biosci (Elite Ed).;4:23-40). Nonsteroidal anti-inflammatory drugs used off label to treat pain in endometriosis reduce the synthesis of a range of prostaglandins.

Therefore, ideally an endometriosis drug would combine some or all of these features:
- inhibit local E2 production without interfering with ovarian estrogen production, thereby leaving systemic estrogen levels in premenopausal patients intact
- increase local progesterone without interfering with systemic progesterone levels thereby leaving the women's natural menstrual cycle unchanged
- reduce inflammation by modulating the ratio of local prostaglandins towards those with predominant anti-inflammatory activity

AKR1C3 is an aldo-keto reductase strongly expressed in endometriotic lesions and only marginally detectable in the ovary (Smuc et al. (2009). Mol Cell Endocrinol.; 301(1-2):59-64). AKR1C3, also known as 17ß-hydroxysteroid dehydrogenase type 5 (17ßHSD5), catalyzes the conversion of estrone (weak estrogenic activity) to estradiol (potent estrogenic activity), conversion of progesterone (potent anti-estrogenic activity) to 20-alpha-hydroxyprogesterone (weak anti-estrogenic activity) and the conversion of androstenedione to testosterone (which can be converted to estradiol by aromatase) (Labrie et al. (2001). Front Neuroendocrinol.; 22(3):185-212). In a concerted action with CYP19A1 (aromatase), AKR1C3 is expected to be a key enzyme in local E2 production in endometriotic lesions, generating a pro-estrogenic environment, thereby stimulating proliferation in estrogen-sensitive endometriotic cells. Inhibition of AKR1C3 should therefore result in decreased local E2 levels and thereby decreased proliferation of endometriotic lesions. Effects on ovarian estrogen production are not expected, since AKR1C3 is only marginally expressed in the ovary and 17βHSD1 is the dominant ovarian hydroxysteroid dehydrogenase.

Beside its 17β-hydroxysteroid dehydrogenase activity, ARK1C3 is a prostaglandin (PG) F2α synthase (Byrns et al. (2010). J Steroid Biochem Mol Biol.; 118(3):177-187). AKR1C3 is capable to catalyze the conversion of PGH2 to PGF2α and PGD2 to 11β-PGF2, both known to stimulate inflammation and proliferation. In the absence of AKR1C3 activity, PGD2 (instead of being converted to PGF2), spontaneously dehydrates and rearranges to form anti-proliferative and anti-inflammatory PGJ2 isomers, including 15d-PGJ2. 15d-PGJ2 is a potent agonist for the nuclear orphan peroxisome proliferator-activated receptor γ (PPARγ) and postulated to be involved in the resolution of inflammation. 15d-PGJ2 also directly reacts with cysteine residues in other proteins, including NF-λB resulting in loss of transcriptional activity, thereby again reducing inflammation and proliferation. 15d-PGJ2 has been shown to inhibit the secretion of pro-inflammatory cytokines from macrophages and to inhibit the production of chemokines in vitro. In addition 15d-PGJ2 has been shown to inhibit the inflammatory hypernociception in rats, indicating an upside potential of the AKR1C3 inhibitor in the treatment of endometriotic pain (Napimoga et al. (2008). J Pharmacol Exp Ther.; 324(1):313-321).

A recent publication reported experience with a different AKR1C3 inhibitor (Loriot et al. (2014). Invest New Drugs., Oct;32(5):995-1004) in patients with castration-resistant prostate cancer. No safety concerns were reported. The target of lowering plasma prostate specific antigen by ≥50% was not achieved and the trial was terminated early. Given the gender differences in sex steroid metabolism the applicability of findings in elderly men to premenopausal women is uncertain.

Therefore, AKR1C3 inhibition is considered to be a highly innovative combination of an anti-inflammatory approach with a local anti-hormonal concept. Inflammation, pain and proliferation are targeted in endometriosis with this unique mechanism of action. The aldo-keto reductase 1C3 (AKR1C3; synonyms: type 5 17β-hydroxysteroid dehydrogenase or prostaglandin F synthase) is a multifunctional enzyme and catalyses, among other processes, the reduction of 4-androstene-3,17-dione (a weak androgen) to testosterone (a potent androgen) and of oestrone (a weak oestrogen) to 17β-oestradiol (a strong oestrogen). In addition, the reduction of prostaglandin (PG) H2 to PGF2α and PGD2 to 9α,11β-PGF2 is inhibited (Penning et. al., 2006, Molecular and Cellular Endocrinology 248(1-2), 182 -191).

Steroidal ARK1C3 inhibitors have been described in the applications WO 20140009274, WO2014128108 and WO 2013045407. These applications also disclose that in the case of oral administration, the amount of compound is about 0.01 to 100 mg/kg of body weight per day which corresponds to a single dose of 0.7 to 7000 mg/70 kg body weight per day for women body weight per day. The data obtained with a concentration of 30 mg/kg daily of AKR1C3 inhibitor in a marmoset endometriosis model clearly show that hardly any lesions could be found on the bladder and on/ in the uterus of the treatment group when compared with the vehicle group (WO 2013045407). In another study also employing the marmoset endometriosis model the AKR1C3 inhibitor was tested in a lower dose (0.2 mg/kg and 0.8 mg/kg, given daily). A reduction in lesion load could be demonstrated in the groups treated with the AKR1C3 inhibitor when compared to the vehicle group (http://www.investor.bayer.com/securedl/11809).

Until now there are no data available indicating a safe dose range of the AKR1C3 inhibitor for human use. Therefore there is a medical need to establish such a relevant dose range for the safe use of the compound.

### SUMMARY OF THE INVENTION

A predicted safe daily dose of the AKR1C3 inhibitor could be established in two different set ups, e.g. clinical studies in women and developmental toxicity studies.

Surprisingly the data of the clinical study showed that the AKR1C3 inhibitor is well tolerated up to dose of of 60 mg twice daily. In addition it was discovered that an area of increased concern for reproductive or developmental toxicity in humans might be expected at about > 15 mg daily dose and of decreased concern at about ≤3 mg daily dose. Thus combining the results of the the clinical studies and the reprotoxicity studies, a safe dose range for women can be established between 0.7 mg - 120 mg / 70 kg body weight per day for women with varing degree of preference.

Laboratory tests (such as hematology, clinical chemistry, coagulation and urine analysis) performed in clinical studies revealed isolated, asymptomatic transient increases in serum bilirubin:
- Up to 30 mg once daily / 70 kg body weight for women no changes in plasma bilirubin were observed upon single and repeat dosing up to 14 days.
- At 90 mg / 70 kg body weight for women once daily (postmenopausal women; 2 weeks of treatment) and 60mg once daily (premenopausal women; 4 weeks of treatment) increases in plasma bilirubin were observed in a small fraction of subjects exposed to the test substance with a trend to return to baseline during the second half of the treatment interval of 2 or 4 weeks, respectively.
- At 60 mg twice daily / 70 kg body weight for women increase in plasma bilirubin was observed in a significant fraction of subjects exposed to the test substance for 14 or 28 days.

After administration of a single dose of 240 mg / 70 kg body weight for women of the AKR1C3 inhibitor gastrointestinal symptoms (mild to moderate nausea, vomiting and/or diarrhea) and an increase in bilirubin were detected (without changes in liver enzymes) within 24 hours post dosing and returned to concentrations below ULN at 48 hours after dosing. These findings were fully reversible and are monitorable.

The increase in total serum bilirubin detected were exclusively isolated i.e. not associated with increases in serum liver enzyme concentrations such as plasma aminotransferase concentrations and alkaline phosphatase concentration and accordingly no signs of drug-induced liver injury (DILI) have been detected. Both unconjugated and the conjugated forms of bilirubin were found to be increased in serum. In addition there was no indication for hemolysis.

This finding is in line with the assumption that it is caused by interaction of of the AKR1C3 inhibitor with several transporters involved in transport of bilirubin from the bloodstream into the hepatocyte and from the hepatocyte into the bile duct.

Genetic variants of bilirubin transporters in the liver are well described (e.g. Morbus Gilbert-Meulengracht, Dubin-Johnson and Rotor syndrome). Carriers of these genetic variants are exposed to increased concentrations of serum bilirubin lifelong (usually less than 3 mg/dl) without apparent increase in mortality or morbidity.

To further establish a safe dose the risk assessment on reproductive and developmental toxicities has to be assessed. In the newly analyzed studies assessing the embryo-fetal development in rats and rabbits after exposure to the AKR1C3-inhibitor *N*-{[17-(5-Fluoropyridin-3-yl)estra-1,3,5(10),16-tetraen-3-yl]carbonyl}-*N*-methyl-*β*-alanine findings pointing to a dose-dependent developmental toxicity were observed:
- In the respective rat study developmental toxicity was noted in rat foetuses when pregnant rats were exposed to the high dose of the compound (60 mg/kg). The effects included malformations (= severe foetal structural abnormalities). When comparing the systemic exposure (AUCunbound) achieved in rats at 17 mg/kg (NOAEL = no-observed-adverse-effect level) to the expected clinical human exposure after administration of the compound ***N***-{[17-(5-Fluoropyridin-3-yl)estra-1,3,5(10),16-tetraen-3-yl]carbonyl}-*N*-methyl-*β*-alanine twice daily 60 mg/day, once daily 30 mg/day and once daily 3 mg/day an approximately 2-fold, 15-fold or 122-fold higher exposure was achieved, respectively (Table 2).
- In the corresponding rabbit embryo-fetal development study no compound-related malformations were revealed. However, the incidence of some foetal structural variations (= mild foetal structural abnormalities) was increased at the mid dose of the compound (20 mg/kg and higher). When comparing the systemic exposure (AUCunbound) achieved in rabbits at 5 mg/kg (NOAEL) to the expected clinical human exposure after administration of the comound *N*-{[17-(5-Fluoropyridin-3-yl)estra-1,3,5(10),16-tetraen-3-yl]carbonyl}-*N-*methyl-*β*-alanine twice daily 60 mg/day, once daily 30 mg/day and once daily 3 mg/day an approximately 0.3-fold, 2-fold or 17-fold higher exposure was achieved, respectively.

Comparison of systemic drug exposure at the NOAEL for the reproductive or developmental toxicity in the test species to that in humans at the maximum recommended dose is a critical determination (refer to "FDA 's Guidance for Industry Reproductive and Developmental Toxicities -Integrating Study Results to Assess Concerns, September 2011"). According to that regulatory guidance, there is increased concern for reproductive or developmental toxicity in humans for relative exposure ratios (animal: human) that are <10 and decreased concern for exposure ratios >25.

Applying these recommendations to the non-clinical studies assessing the embryo-fetal development in rats and rabbits after exposure to the AKR1C3-inhibitor ***N***-{[17-(5-Fluoropyridin-3-yl)estra-1,3,5(10),16-tetraen-3-yl]carbonyl}-*N*-methyl-*β*-alanine, the most preferred target dose of the AKR1C3-inhibitor to be further developed in the clinical program should be restricted to an upper limit of once daily 15 mg/day, corresponding to a >25 exposure ratio for induction of malformations (compared to the human exposure) in both tested animal species.

In conclusion, the AKR1C3 inhibitor was well tolerated up to a single dose of 120 mg / 70 kg body weight for women or 60 mg twice daily.

Therefore it is an object of the present invention to provide a low-dosed oral dosage form of the AKR1C3 inhibitor *N*-{[17-(5-Fluoropyridin-3-yl)estra-1,3,5(10),16.-tetraen-3-yl]carbonyl}-*N*-methyl-*β*-alanine which is effective in the treatment and/or prophylaxis diseases, especially endometriosis, polycystic ovary syndrome and atopic dermatitis.

### FIGURES

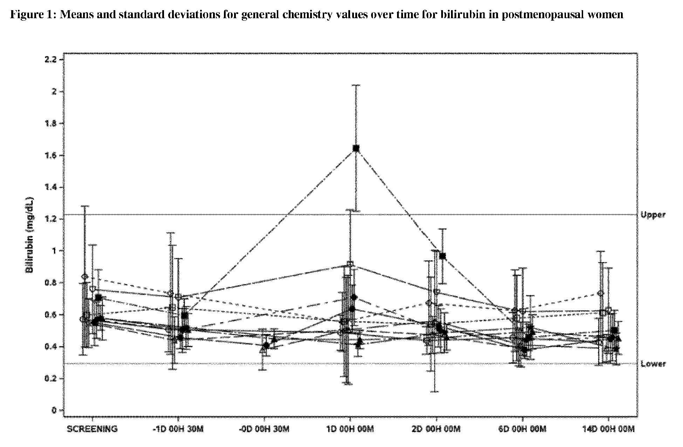

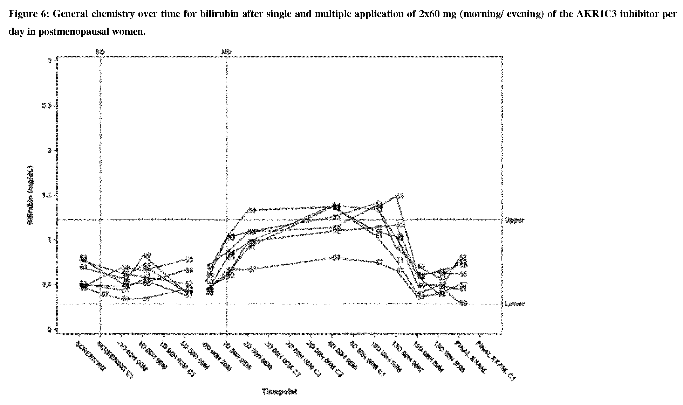

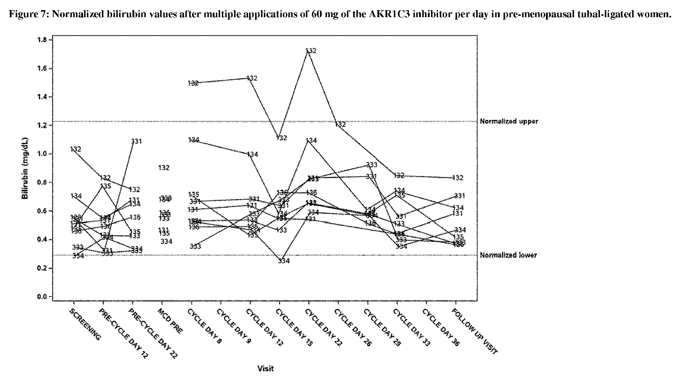

**Figure 1****: Means and standard deviations for general chemistry values over time for bilirubin in postmenopausal women**
   The graph describes the time course of bilirubin concentration in blood of healthy volunteers (postmenopausal women) after single dose of 1 mg (empty circle), 3 mg (empty diamond), 10 mg (empty square), 30 mg (empty star), placebo (filled star) all formulated as oral drinking solution and 30 mg (empty triangle), 60 mg (empty polygon), 90 mg (filled circle), 120 mg (filled diamond), 240 mg (filled square), placebo (filled triangle) all formulated as tablets. Values are depicted for the following time points: At screening, one day before first treatment, at the day of first treatment before first intake, on days 1, 2, 6, 14 after intake. In addition the upper and lower limits of norm for bilirubin are reflected.

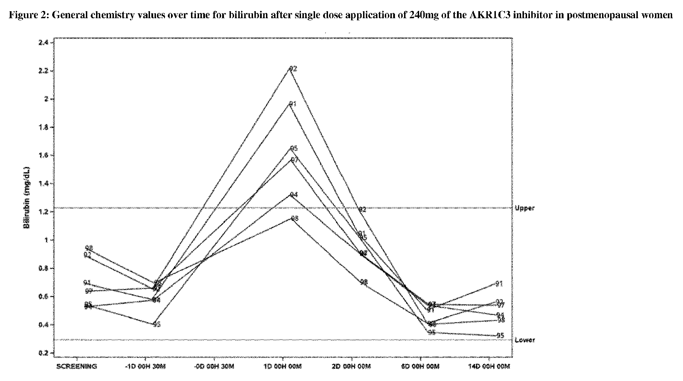

**Figure 2****: General chemistry values over time for bilirubin after single dose application of 240 mg of the AKR1C3 inhibitorin postmenopausal women**
   The graph describes the time course of the individual bilirubin concentration in blood of 6 healthy volunteers (postmenopausal women) after single dose of 120 mg. Individuals are referenced with the number next to the graph. Values are depicted for the following time points: At screening, one day before first treatment, at the day of first treatment before first intake, on days 1, 2, 6, 14 after intake. In addition the upper and lower limits of norm for bilirubin are reflected.

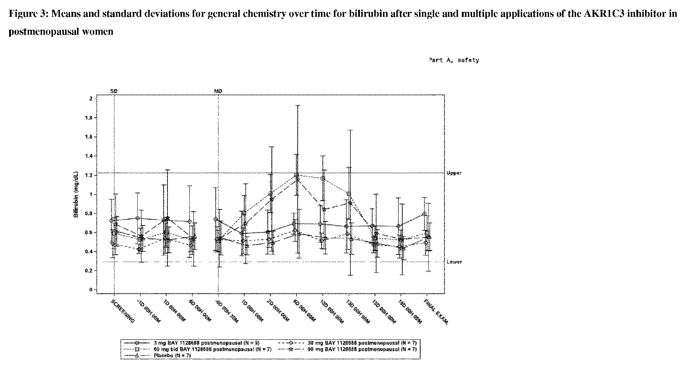

**Figure 3****: Means and standard deviations for general chemistry over time for bilirubin after single and multiple applications of the AKR1C3 inhibitor in postmenopausal women**
   The graph describes the time course of bilirubin concentration in blood of healthy volunteers (postmenopausal women) after single and multiple applications of doses of 3 mg (empty circle), 30 mg (empty diamond), 120 mg (empty square), 90 mg (empty star) and placebo (empty triangle) given in tablet form daily. Values are depicted for the following time points: At screening, one day before first treatment, at the day of first treatment before first intake and after first intake on days 1 and 6 after single and multiple dose application and in addition for multiple dose application at days 10, 13, 15, 19 and at final examination. In addition the upper and lower limits of norm for bilirubin are reflected.

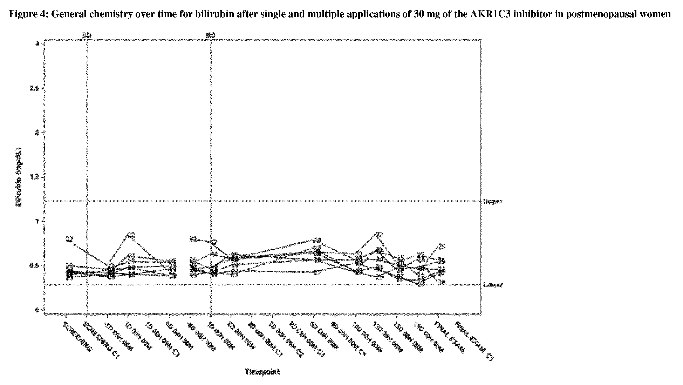

**Figure 4****: General chemistry over time for bilirubin after single and multiple applications of 30 mg of the AKR1C3 inhibitor in postmenopausal women**
   The graph describes the time course of bilirubin concentration in blood of 7 healthy volunteers (postmenopausal women) after single and multiple applications of 30 mg of the AKR1C3 inhibitor given in tablet form daily. Individuals are referenced with the number next to the graph. Values are depicted for the following time points: At screening, one day before first treatment, at the day of first treatment before first intake and after first intake on days 1 and 6 after single and multiple dose application and in addition for multiple dose application at days 10, 13, 15, 19 and at final examination. In addition the upper and lower limits of norm for bilirubin are reflected.

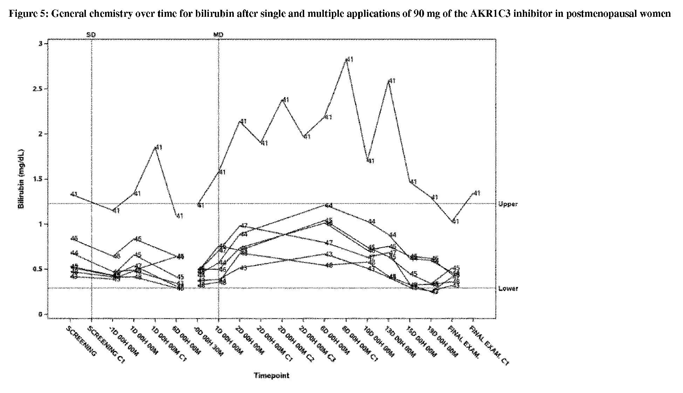

**Figure 5****: General chemistry over time for bilirubin after single and multiple applications of 90 mg of the AKR1C3 inhibitor in postmenopausal women**
   The graph describes the time course of bilirubin concentration in blood of 7 healthy volunteers (postmenopausal women) after single and multiple applications of 90 mg of the AKR1C3 inhibitor given in tablet form daily. Individuals are referenced with the number next to the graph. Values are depicted for the following time points: At screening, one day before first treatment, at the day of first treatment before first intake and after first intake on days 1 and 6 after single and multiple dose application and in addition for multiple dose application at days 10, 13, 15, 19 and at final examination. In addition the upper and lower limits of norm for bilirubin are reflected. For the individual with the reference number 41 increased numbers of values over the same time course are depicted
**Figure 6****: General chemistry over time for bilirubin after single and multiple application of 2x60 mg (morning/ evening) of the AKR1C3 inhibitor per day in postmenopausal women.**
   The graph describes the time course of bilirubin concentration in blood of 7 healthy volunteers (postmenopausal women) after single and multiple applications of 2x60 mg of the AKR1C3 inhibitor given in tablet form daily. Individuals are referenced with the number next to the graph. Values are depicted for the following time points: At screening, one day before first treatment, at the day of first treatment before first intake and after first intake on days 1 and 6 after single and multiple dose application and in addition for multiple dose application at days 10, 13, 15, 19 and at final examination. In addition the upper and lower limits of norm for bilirubin are reflected
**Figure 7****: Normalized bilirubin values after multiple applications of 60 mg of the AKR1C3 inhibitor per day in pre-menopausal tubal-ligated women.**
   The graph describes the time course of normalized bilirubin values in blood of 9 healthy volunteers (pre-menopausal tubal-ligated women) after application of 60 mg of the AKR1C3 inhibitor given in tablet form daily over the time course of 4 weeks. Individuals are referenced with the number next to the graph. Values are depicted for the following time points: At screening, 12 and 22 days pre-cycle, beginning of cycle, on cycle days 8, 9, 12, 15, 22, 26, 29 and 33 as well as on the follow up visit. In addition the normalized upper and lower limits of normal for bilirubin are reflected.

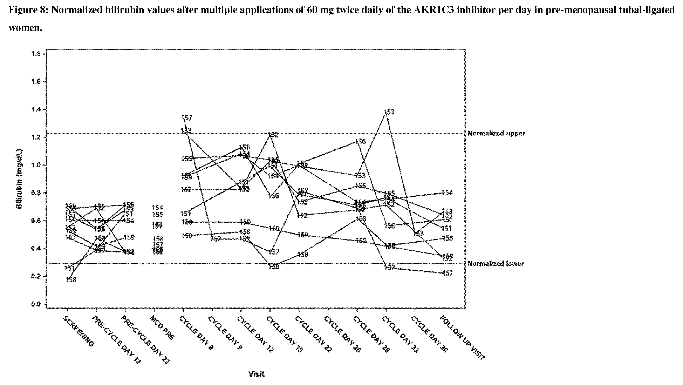

**Figure 8****: Normalized bilirubin values after multiple applications of 60 mg twice daily of the AKR1C3 inhibitor per day in pre-menopausal tubal-ligated women.**
   The graph describes the time course of normalized bilirubin values in blood of 9 healthy volunteers (pre-menopausal tubal-ligated women) after application of 60 mg twice daily of the AKR1C3 inhibitor given in tablet form (60 mg in the morning and 60 mg in the evening) daily over the time course of 4 weeks. Individuals are referenced with the number next to the graph. Values are depicted for the following time points: At screening, 12 and 22 days pre-cycle, beginning of cycle, on cycle days 8, 9, 12, 15, 22, 26, 29 and 33 as well as on the follow up visit. In addition the normalized upper and lower limits of normal for bilirubin are reflected.

### DETAILED DESCRIPTION OF THE INVENTION

**DEFINITIONS**

The present invention is directed to an oral dosage form comprising doses up to 120 mg *N*-{[17-(5-Fluoropyridin-3-yl)estra-1,3,5(10),16-tetraen-3-yl]carbonyl}-*N*-methyl-*β*-alanine. The synthesis of the intermediates and of the compound, the purification as well as a dose range and the efficacy of the AKR1C3 inhibitor has been dislosed in the application WO 2013/045407, this subject matter is and herewith included into the application.

As discussed supra, the dosage form of the invention is suitable to be used already as the initial treatment of the above-mentioned conditions, in particular for the prevention, treatment or alleviation of endometriosis, polycystic ovary syndrome and atopic dermatitis. Accordingly, in an interesting embodiment, the dosage form of the invention is administered to a woman who suffers from the above cited diseases.

For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

In the present context, the term "oral dosage form" generally refers to tablets (both swallowable-only and chewable forms), capsules, granules, granules enclosed in sachets and pills. Hence, the dosage form of the invention may be in the form of a tablet, capsule, gelcap, granule, sachet or a pill. In a preferred embodiment of the invention, the dosage form is in the form of a tablet or a capsule, in particular in the form of a tablet. This is not limited to other formulations achieving similar exposures.

Based upon standard laboratory techniques known to evaluate compounds useful for the treatment of endometriosis, polycystic ovary syndrome and atopic dermatitis, by standard toxicity tests and by standard pharmacological assays for the determination of treatment of the conditions identified above in mammals, and by comparison of these results with the results of known active ingredients or medicaments that are used to treat these conditions, the effective dosage of the compounds of the present invention can readily be determined for treatment of each desired indication. The amount of the active ingredient to be administered in the treatment of one of these conditions can vary within the dose range claimed in this invention according to such considerations as the particular compound and dosage unit employed the mode of administration, the period of treatment, the age and sex of the patient treated, and the nature and extent of the condition treated.

### Clinical experiments

The following table 1 gives an overview of the relevant clinical studies conducted with the AKR1C3 inhibitor and includes data on population, administration route and tested dose ranges.

**Table 1: Relevant clinical studies**

| **Type of Study** | **Population** | **Method of Administration** | **Doses** |
|---|---|---|---|
| Single dose study #1 | Postmenopausal women [healthy volunteers] | Oral (liquid) | 0 mg, 1 mg, 3 mg, 10 mg, 30 mg (single dose administrations) |
| Single dose study #1 | Postmenopausal women [healthy volunteers] | Oral (tablet) | 30 mg, 60 mg, 90 mg, 120 mg, 240 mg (single dose administrations) |
| Multiple dose study, Part A #2 | Postmenopausal women [healthy volunteers] | Oral (tablet) | 0 mg once or twice daily, 3 mg once daily, 30 mg once daily and 90 mg once daily and 60 mg twice daily |
| Multiple dose study, Part B #2 | Premenopausal women [healthy volunteers] | Oral (tablet) | 60 mg once daily and 60 mg twice daily |

In the following the methods used and the results gained will be described in detail for each of the above mentioned clinical studies:
Single dose study 1: Healthy postmenopausal women received single administration of drinking solution (liquid formulation) containing either 0 mg (control), 1 mg, 3 mg, 10 mg or 30 mg of the AKR1C3 inhibitor or tablets containing 30 mg, 60 mg, 90 mg, 120 mg, 240 mg of the AKR1C3 inhibitor. Plasma bilirubin levels were assessed prior to dosing and 1, 2, 6 and 14 days after drug administration. Plasma bilirubin concentrations above the upper limit of normal were detected in 5 of 6 subjects who received a single dose of 240 mg / 70 kg body weight per day for women the of AKR1C3 inhibitor (see figures 1 and 2).
Multiple dose study 2 Part A: Healthy postmenopausal women received tablets containing 3 mg (once daily). 30 mg (once daily), 90 mg (once daily) or 60 mg (twice daily) per 70 kg body weight for women of the AKR1C3 inhibitor over 14 days. Plasma bilirubin levels were assessed prior to dosing and 1, 2, 6, 10, 13, 15, 19 days after drug administration and at final examination. Plasma bilirubin concentrations above the upper limit of normal were detected in 1 of 7 subjects who received 90 mg once daily and in 5 of 7 subjects who received 60 mg twice daily (see figures 3 to 6).
Multiple dose study 2 Part B: Healthy premenopausal women received tablets containing 60 mg once daily or 60 mg twice daily of the AKR1C3 inhibitor over 28 days. Plasma bilirubin levels were assessed prior to dosing and on cycle days 8, 12, 15, 22, 29, 33 after start of drug administration and at the follow up examinations. Plasma bilirubin concentrations above the upper limit of normal were detected in 1 of 9 subjects who received 60 mg once daily and in 2 of 9 subjects who received 60 mg twice daily (see figures 7 and 8).

In all of the above mentioned studies the observed increases in bilirubin were reversible. Accordingly, after cessation of treatment plasma bilirubin concentrations returned to a range of concentrations similar to those detected pre treatment.

In summary, the above listed findings indicate a dose-dependent increase in plasma bilirubin upon exposure to the test substance with the following observations:
- Up to 30 mg once daily / 70 kg body weight per day for women woman no changes in plasma bilirubin were observed upon single and repeat dosing up to 14 days.
- At 90 mg once daily / 70 kg body weight per day for women woman (postmenopausal women; 2 weeks of treatment) and 60 mg once daily / 70 kg body weight per day for women woman (premenopausal women; 4 weeks of treatment) increases in plasma bilirubin were observed in a small fraction of subjects exposed to the test substance with a trend to return to baseline during the second half of the treatment interval of 2 or 4 weeks, respectively.
- At 60 mg twice daily / 70 kg body weight per day for women woman increase in plasma bilirubin was observed in a significant fraction of subjects exposed to the test substance for 14 or 28 days.

### Reprotoxicity studies:

The reproduction toxicity studies with N-{[17-(5-Fluoropyridin-3-yl)estra-1,3,5(10),16-tetraen-3-yl]carbonyl}-N-methyl-β-alanine assessing the potential of developmental toxicity were conducted in rats and rabbits and are summarized in the following. The safety margins (= multiple of exposure, MoE) to the no-observed-adverse-effect level (NOAEL) are given in table 2. The experimental details are described in example 3.

In the studies assessing the embryo-fetal development in rats and rabbits findings pointing to developmental toxicity were observed. In the respective rat study in which the doses of 5, 17 and 60 mg/kg/day of the compound *N*-{[17-(5-Fluoropyridin-3-yl)estra-1,3,5(10),16-tetraen-3-yl]carbonyl}-*N*-methyl-*β*-alanine were applied, developmental toxicity was noted in rat foetuses when pregnant rats were exposed to the high dose of the compound *N*-{[17-(5-Fluoropyridin-3-yl)estra-1,3,5(10),16-tetraen-3-yl]carbonyl}-*N*-methyl-*β*-alanine (60 mg/kg). The effects included malformations (= severe foetal structural abnormalities) occurring at a low incidence and variations noted at a higher incidence compared to the concurrent and historical control. The NOAEL for developmental toxicity in rats is consequently the mid dose of 17 mg/kg. When comparing the systemic exposure (AUCunbound) achieved in rats at 17 mg/kg to the expected clinical exposure after administration of twice daily 60 mg/day, once daily 30 mg/day and once daily 3 mg/day an approximately 2-fold, 15-fold or 122-fold higher exposure, respectively, was achieved in pregnant rats at the NOAEL for developmental toxicity of 17 mg/kg.

In the corresponding rabbit embryo-fetal development study in which the doses of 5, 20 and 70 mg/kg of the compound *N*-{[17-(5-Fluoropyridin-3-yl) estra-1,3,5(10),16-tetraen-3-yl]carbonyl}-*N*-methyl-*β-*alanine were administered, no compound-related malformations were revealed. However, the incidence of some foetal structural variations (= mild foetal structural abnormalities) was increased at the mid dose of 20 mg/kg and higher. The NOAEL with regard to developmental toxicity in rabbits is thus the low dose of 5 mg/kg of *N*-{[17-(5-Fluoropyridin-3-yl)estra-1,3,5(10),16-tetraen-3-yl]carbonyl}-*N*-methyl-*β*-alanine. When comparing the systemic exposure (AUCunbound) achieved in rabbits at 5 mg/kg of the compound to the expected clinical exposure after administration of twice daily 60mg/day, once daily 30 mg/day and once daily 3 mg/day of the compound *N*-{[17-(5-Fluoropyridin-3-yl)estra-1,3,5(10), 16-tetraen-3-yl]carbonyl}-*N*-methyl-,*β*-alanine an approximately 0.3-fold, 2-fold or 17-fold higher exposure was achieved, respectively.

**Table 2: Preclinical safety margins**

| **Preclinical safety margins based** on **AUCunbound:** | | | | | |
|---|---|---|---|---|---|
| **Type of Study** | **Doses** [**mg**/**kg**] | **NOAEL for dev**. **toxicity** | **Safety margin (MoE) to NOAEL for developmental toxicity at** | | |
| | | | **2x 60 mg/day** | **30 mg/day** | **3 mg/day** |
| EFD, rats | 0, 5, 17, 60 | 17 mg/kg | **∼ 2** | ∼ 15 | **∼ 122** |
| EFD, rabbits | 0, 5, 20, 70 | 5 mg/kg | ∼ 0.3 | ∼ 2 | ∼ 17 |

| | | | | | |
|---|---|---|---|---|---|
| NOAEL = No observed effect level MoE = Multiples of systemic exposure EFD = Embryonic development study | | | | | |

To summarize the data of the clinical study showed that the AKR1C3 inhibitor N-{[17-(5-Fluoropyridin-3-yl)estra-1,3,5(10), 16-tetraen-3-yl]carbonyl}-N-methyl-β-alanine is well tolerated up to a dose of 60 mg twice daily. In addition it was discovered that an area of increased concern for reproductive or developmental toxicity in humans might be expected at about > 15 mg daily dose and of decreased concern at about ≤3 mg daily dose. Thus combining the results of the clinical studies and the reprotoxicity studies, a safe dose range for women can be established between 0.7 mg - 120 mg / 70 kg body weight per day for women with varing degree of preference.

Therefore an object of the present invention is an oral dosage form comprising up to 120 mg *N*-{[17-(5-Fluoropyridin-3-yl)estra-1,3,5(10),16-tetraen-3-yl]carbonyl}-*N*-methyl-*β*-alanine. Another object of the present invention is said oral dosage form whereby the dosage is 120 mg. Another object of the present invention is said oral dosage form whereby the dosage is 60 mg. Another object of the present invention is said oral dosage form whereby the dosage is 30 mg. Another object of the present invention is said oral dosage form whereby the dosage is 15 mg. Yet another object of the present invention is said oral dosage form whereby the dosage is 3 mg. Yet another object of the present invention is said oral dosage form whereby the dosage is 1 mg. Another object of the present invention is said dosage form whereby the dosage form is a tablet. The tablet can be film-coated. The dosage form can also be liquid. As disclosed in the application WO 2013045407 the compound N-{[17-(5-Fluoropyridin-3-yl)estra-1,3,5(10), 16-tetraen-3-yl]carbonyl}-*N*-methyl-*β*-alanine is a potent AKR1C3 inhibitor. As dicussed supra the AKR1C3 inhibition is considered to be a highly innovative combination of an anti-inflammatory approach with a local anti-hormonal concept. Therefore the use of said dosage form as a medicament is also object of the present invention.

### Endometriosis:

Endometriosis can be defined as the presence of endometrium-like tissue ('endometriotic lesions') outside the lining of the uterus, which induces a chronic inflammatory reaction (Dunselman, GA, N Vermeulen et al. ESHRE guideline: management of women with endometriosis. Hum Reprod. 2014;29:400-12). With a prevalence of approximately 5-10% of the female population, endometriosis is estimated to affect 176 million women worldwide (Adamson, GD, S Kennedy et al. Creating solutions in endometriosis: global collaboration through the World Endometriosis Research Foundation. Journal of Endometriosis 2010;2:3-6). In key markets, there are estimated to be 10.1 million patients with the disease (Bernuit, D, AD Ebert et al. Female perspectives on endometriosis: findings from the uterine bleeding and pain women's research study. Journal of Endometriosis 2011;3:73-85). Endometriosis can have a severe impact on women's lives. For many, the symptoms are so bad that they are bedridden for an average of 18 days a year (Kjerulff, KH, BA Erickson et al. Chronic gynecological conditions reported by US women: findings from the National Health Interview Survey, 1984 to 1992. Am J Public Health. 1996;86:195-9). There is often a considerable delay between the onset of symptoms and diagnosis, (Nnoaham, KE, L Hummelshoj et al. Impact of endometriosis on quality of life and work productivity: a multicenter study across ten countries. Fertil Steril. 2011;96:366-373 e8) and definitive diagnosis requires laparoscopic visualization of the internal structures of the pelvis, ideally with histological confirmation. Even in developed countries like Germany and Austria where laparoscopy is widely available, the diagnostic delay can be more than 10 years (Hudelist, G, N Fritzer et al. Diagnostic delay for endometriosis in Austria and Germany: causes and possible consequences. Hum Reprod. 2012;27:3412-6).

Endometriosis can appear as lesions of variable size on the peritoneum, endometriotic cysts on the ovaries (endometriomas), and may form adhesions between organs. Lesions may be superficial or deeply infiltrating (penetrate 5 mm or more) and often infiltrate the rectovaginal space. Endometriosis can also develop on other structures within the pelvis, such as the bladder, ureters or fallopian tubes. Furthermore, endometriosis can appear in the myometrium of the uterus, so-called adenomyosis. Rarely, organs outside the pelvis are involved e.g. pericardium, pleura, brain etc.

Lesions are hormone-responsive, and may haemorrhage during menstruation. Women with endometriosis may suffer from chronic, cyclic pelvic pain (dysmenorrhea) and/or non-cyclic pelvic pain, have impaired fertility and experience pain during intercourse (dyspareunia). Of these, dysmenorrhea is often reported as the most common symptom (Sinaii, N, K Plumb et al. Differences in characteristics among 1,000 women with endometriosis based on extent of disease. Fertil Steril. 2008;89:538-45). Other symptoms may include gastrointestinal disturbances, urinary problems and fatigue. The most accepted theory for how endometriosis arises is that during menstruation, endometrial tissue is shed through the fallopian tubes (so-called 'retrograde menstruation') and implants on the peritoneal surfaces (Giudice, LC. Clinical practice. Endometriosis. N Engl J Med. 2010;362:2389-98). However, this theory does not explain the manifestation of endometriosis before the menarche or the appearance of lesions outside the pelvis, and other processes may contribute to the pathogenesis of endometriosis.

According to guidelines from the American Society for Reproductive Medicine (ASRM) "endometriosis should be viewed as a chronic disease that requires a life-long management plan with the goal of maximizing the use of medical treatment and avoiding repeated surgical procedures." (Practice Committee of the American Society for Reproductive Medicine. Treatment of pelvic pain associated with endometriosis: a committee opinion. Fertil Steril. 2014;101:927-35). Prior to receiving a laparoscopic diagnosis, women with endometriosis are commonly treated with non-steroidal anti-inflammatory drugs (NSAIDs), opioids and/or empirically with combined oral contraceptives (COCs) or progestins (Cea-Soriano, LGR, L. A., R Schulze-Rath et al. Treatment patterns of endometriosis in the health improvement network primary care database. 'Presented at' Reproductive Sciences 2015;22:115A. Presented at the 62nd Annual Scientific Meeting of the Society for Gynecologic Investigation (SGI 2014), 25-28 March 2014, San Francisco, CA, USA). The use of COCs is off-label, while a few progestins have been licensed in some countries for this indication. Visanne® is the only progestin that has been specifically developed for the treatment of this disease, and was the first medical therapy approved for endometriosis in the EU since the introduction of gonadotropin-releasing hormone (GnRH) analogues in 1989.

Although administration of COCs reduces endogenous estradiol levels, little is known about the long-term effects of exogenous estrogen on disease progression. It has been suggested that the use of COCs may facilitate the implantation and growth of ectopic endometrial cells, so increasing the risk of endometriosis (Parazzini, F, M Ferraroni et al. Contraceptive methods and risk of pelvic endometriosis. Contraception, 1994;49:47-55). Thus, the use of COCs could allow disease progression while masking the symptoms of disease. Furthermore, women with endometriosis frequently find COCs or progestins unsuitable due to side effects or ineffectiveness, although little data are available on the estimated failure rates of these agents (Kappou, D, M Matalliotakis et al. Medical treatments for endometriosis. Minerva Ginecol. 2010;62:415-32, and Seracchioli, R, M Mabrouk et al.). Post-operative use of oral contraceptive pills for prevention of anatomical relapse or symptom-recurrence after conservative surgery for endometriosis. Hum Reprod. 2009;24:2729-35). However, there are no alternative, long-term medical therapies available. GnRH analogues are limited to short-term use due to hypo-estrogenic side effects, and danazol (an androgen) and gestrinone (an anti-progestin) are rarely prescribed. Surgical procedures for the treatment of endometriosis can be ineffective. Recurrence rates of 40-50% at 5 years have been reported, with many patients undertaking repeated surgery (Guo, SW. Recurrence of endometriosis and its control. Hum Reprod Update. 2009;15:441-61). Even hysterectomy with removal of both ovaries is not necessarily a definitive treatment for this disease; recurrent symptoms of endometriosis have been reported in 10% of women undergoing this procedure (Namnoum, AB, TN Hickman et al. Incidence of symptom recurrence after hysterectomy for endometriosis. Fertil Steril. 1995;64:898-902).

Since Inflammation, pain and proliferation are targeted in endometriosis with the unique mechanism, of AKR1C3 inhibition, the use of said dosage form for the prevention, treatment or alleviation of endometriosis is an object of the present invention.

### Polycystic ovary syndrome:

Polycystic ovary syndrome (PCOS) is a common endocrine system disorder among women of reproductive age. Women with PCOS may have enlarged ovaries that contain small collections of fluid - called follicles - located in each ovary as seen during an ultrasound exam. Infrequent or prolonged menstrual periods, excess hair growth, acne, and obesity can all occur in women with polycystic ovary syndrome. In adolescents, infrequent or absent menstruation may raise suspicion for the condition. The exact cause of polycystic ovary syndrome is unknown. Early diagnosis and treatment along with weight loss may reduce the risk of long-term complications, such as type 2 diabetes and heart disease. (http://www.mayoclinic.org/diseases-conditions/pcos/basics/definition/con-20028841). Recent studies also demonstrat a significantly increased risk of non-alcoholic fatty liver disease. PCOS therefore represents a significant health care burden.

Androgen excess is a major driver of adverse metabolic risk. It was demonstrated that with increasing severity of androgen excess also the prevalence of insulin resistance increases (O'Reilly M et al., JCEM 2014). This has been confirmed by others (Pasquali R, JCEM 2016). AKR1C3 is overexpressed in adipose tissue in women with simple obesity and PCOS, where it can activate androstenedione to testosterone (Quinkler et al., J Endocrinol 2004; Wang L et al., JSBMB 2012). Thus an inhibition of AKR1C3 is expected to be advantageous for the treatment of PCOS.

Therefore the use of said dosage form for the prevention, treatment or alleviation of PCOS is an object of the present invention.

### Atopic dermatitis:

Atopic dermatitis (AD) is a chronic, pruritic inflammatory skin disease of unknown origin that usually starts in early infancy, but also affects a substantial number of adults. As already discussed supra AKR1C3 has been shown to mediate the metabolism of sex hormones and prostaglandin D2 (PGD2). Mantel et al. (J Invest Dermatol. 2012 Apr; 132(4): 1103-1110) describe AKR1C3 as a lipid mediator that promotes skin inflammation in atopic dermatitis (AD). Both play a role in skin function and pathology. The authors describe that AKR1C3 was found to be upregulated in AD but not in psoriasis lesions compared with non-lesional skin and demonstrate a function for AKR1C3 in differentiation-associated gene regulation and also suggests a role in supporting inflammation in AD.

Therefore the use of said dosage form for the prevention, treatment or alleviation of atopic dermatitis is an object of the present invention.

Also an object of the present invention is a method for preventing, treating or alleviating endometriosis, polycystic ovary syndrome and atopic dermatitis comprising administering a dosage form of the present invention to a woman in need thereof.

### EXAMPLES

### Example 1 - single dose study

Single center, randomized, placebo-controlled, double-blind and parallel group study. For the 30 mg group only, a one sequence cross-over design was applied to estimate the relative bioavailability of a liquid versus a solid formulation. The following single dose administrations were tested in this study:
- Liquid dosage form: 1 mg,3 mg,10 mg, 30 mg of the AKR1C3 inhibitor
- Solid dosage form: 30 mg, 60 mg, 90 mg, 120 mg, 240 mg the AKR1C3 inhibitor

In this study the AKR1C3 inhibitor was administered as a single oral dose. Eight volunteers per treatment group were included. Two volunteers per treatment group were allocated to placebo in a randomized, double-blind fashion in order to assess non-drug related factors in the safety evaluation. To ensure that 8 subjects were treated, during the pre-dose phase at least 1 backup subject had to undergo all necessary measurements to compensate for the risk that a woman had to be excluded immediately before dosing due to safety concerns.

Of 115 subjects (healthy postmenopausal women) screened, 60 subjects with a mean age of 57.6 years (SD 4.7 years) were randomized and treated with single doses of the AKR1C3 inhibitor N-{[17-(5-Fluoropyridin-3-yl)estra-1,3,5(10),16-tetraen-3-yl]carbonyl}-N-methyl-β-alanine or placebo. Forty-six (46, 76.7%) out of 60 treated subjects were treated with the AKR1C3 inhibitor (6 subjects per group at dose levels 1 mg, 3 mg, 10 mg, 30 mg, 120 mg, 240 mg; 5 subjects per group at 60 mg and 90 mg) and 14 subjects (23.3%) received placebo (2 subjects each in the 1 mg, 3 mg, 30 mg, 60 mg, 120 mg and 240 mg groups, and 1 subject each in the 10 mg and 90 mg groups). All 60 treated subjects (100%) completed the study and were included in the statistical analyses of safety (SAF: N=60). One out of 60 treated subjects was not valid for the analysis of pharmacodynamics due to vomiting before two times tmax (PDS: N=59). This same subject was also excluded from the PK analysis of the 46 verum-treated subjects, and all 14 placebo-treated subjects were not valid for the analysis of PK (PKS: N=45).

The AKR1C3 inhibitor was well tolerated up to a single dose of 120 mg with an acceptable safety profile for all doses tested including the highest single dose of 240 mg. Based on the tolerability profile of 240 mg, the single dose of 120 mg is considered the maximum tolerated dose.

Bilirubin was measured employing a standard laboratory kit. The tests were performed according to the instructions as provided by the manufacturer (Beckman coulter kit AU680).

### Example 2 - multiple dose study

The study was performed as an ascending dose study after multiple oral doses of the AKR1C3 inhibitor or placebo in 27 healthy postmenopausal (PM) women, aged 45 to 68 years (Part A) and 18 healthy tubal-ligated (TL) women, aged 18 to 48 (Part B). In addition, a lower dose (3 mg or placebo) was evaluated in 9 healthy postmenopausal women, mainly to characterize pharmacokinetic properties of AKR1C3 inhibitor before use in patient studies.

The study consists of two parts: Part A was performed in healthy postmenopausal women (randomized within each dose level and placebo-controlled), Part B in healthy premenopausal women with tubal-ligation (open label; intraindividual comparison to pre-treatment cycle) with 28 days treatment (i.e. approx. one menstrual cycle). The safety of 30 mg once daily, 90 mg once daily, 60 mg twice/day of the AKR1C3 inhibitor given over 14 days was assessed in postmenopausal women prior to exposing premenopausal women to the corresponding doses of 60 mg once daily, 60 mg twice/day over 28 days.
Part A and Part B were further divided into different periods:
   Part A: screening, pre-dose, treatment period 1 with single dose administration, treatment period 2 with multiple dose administration on 14 consecutive days and follow-up. A wash-out period between treatment period 1 (administration day) and treatment period 2 (first administration) of at least 14 days and not more than 28 days was necessary.
      Women received the study medication in a fasted state in period 1 (single dose) as well as on day 14 of period 2 (multiple dose). On day 1 of period 2 the study medication was given after breakfast to evaluate the food effect.
      Three increasing dosages of the AKR1C3 inhibitor were tested: 30 mg once daily, 90 mg once daily and 60 mg twice daily. In addition, a lower dose 3 mg once daily was evaluated. Nine volunteers per dose level were included into the study; thereof, n = 7 women were randomly assigned to treatment with the respective dose and n = 2 to placebo treatment. The study drug was administered in a double-blind manner within one dose level, i.e. all volunteers of that dose level received the same number of tablets identical in appearance.
   Part B: screening, pre-dose (pre-treatment cycle), treatment period with multiple dose administration on 28 consecutive days and follow-up. In the pre-dose period, hormonal changes during a menstrual cycle including evidence of ovulation were evaluated. Study drug administration started on day 4 of the menstrual bleeding after end of the pre-treatment cycle, the study drug was then be taken on 28 consecutive days, independent of further menstrual bleedings. The pre-treatment cycle can be separated by up to 2 menstrual cycles from the treatment period.

In the pre-treatment cycle as well as in the treatment period regular transvaginal ultrasound and hormone measurements were done to decide upon ovarian activity. In addition, a panel of selected hormones was analyzed daily around the LH peak. To detect this peak, the women had to perform a home urine test (Clearblue Fertility Monitor or comparable device) daily which analyzed LH and estrone-3-glucuronide (E3G).

Uterine bleedings and spottings were documented in a diary daily, starting with the pre-treatment cycle until follow-up. Menstrual effluent (menstrual blood) was collected on a single day on two occasions: a) before treatment and b) on the occurrence of the next menstrual bleeding during or after the treatment period depending on the individual cycle length (treatment was always for 28 days).

Serious treatment-emergent adverse events (TEAEs) were not reported in this clinical study. The occurrence of drug-related TEAEs was 100% at the highest tested dose of AKR1C3 inhibitor (240 mg). The most common drug-related TEAEs in the group of subjects treated with AKR1C3 inhibitor that occurred at a rate greater than placebo were headache (21.7% vs. 0.0%), nausea (15.2% vs. 0.0%), vomiting (13.0% vs. 0.0%), asthenia (13.0% vs. 0.0%), and blood bilirubin increased (13.0% vs. 0.0%). All cases of drug-related asthenia, and blood bilirubin increased occurred in subjects treated with a single dose of 240 mg AKR1C3 inhibitor. Laboratory tests (such as hematology, clinical chemistry, coagulation and urinalysis) revealed no time-related increase in abnormal individual findings and no dose-dependent effect for the tested single doses of AKR1C3 inhibitor except for transient increases in serum bilirubin in the 30 - 240 mg groups, but only in the 240 mg group mean bilirubin values increased above the upper limit of the normal range (ULN) within 24 hours post dosing and returned to concentrations below ULN at 48 hours after dosing. Higher doses of AKR1C3 inhibitor were associated with clinically significant elevations (> 1.5 times above ULN in postmenopausal women) in serum progesterone (primarily after 90, 120 and 240 mg), DOC (primarily after 240 mg) and pregnenolone (primarily after 240 mg).

Total bilirubin concentrations were monitored throughout this study.Bilirubin was measured employing a standard laboratory kit. The tests were performed according to the instructions as provided by the manufacturer (Beckman coulter kit AU680).

The concentration-time courses of all analytes were tabulated separated by treatment. The following statistics were calculated for each of the sampling points: geometric mean, geometric standard deviation (re-transformed standard deviation of the logarithms) and geometric coefficient of variation (CV), arithmetic mean, standard deviation and CV, minimum, median, maximum value and the number of measurements. Means at any time were only calculated if at least 2/3 of the individual data were measured and were above the lower limit of quantification (LLOQ). For the calculation of the mean value a data point below LLOQ was substituted by one half of this limit. In tables showing mean values, where values below LLOQ are included in the calculation of mean values, these means are marked. Individual and geometric mean concentration versus time curves of all analytes (using the actual sampling times for individual plots and the planned sampling times for mean plots) were plotted by study part and treatment using both linear and semi-logarithmic scale.

### Example 3 - preclinical experiments reprotox, description and data

The following table gives an overview on relevant reproduction toxicity studies conducted with the N-{[17-(5-Fluoropyridin-3-yl)estra-1,3,5(10),16-tetraen-3-yl]carbonyl}-N-methyl-β-alanine and includes data on species, administration route and tested dose ranges. Systemic exposure calculated as AUCunbound were estuimated.

**Table 3: Relevant reproduction toxicity studies:**

| **Type of Study** | **Species and [Sex]** | **Method of Administration** | **Doses [mg/kg]** |
|---|---|---|---|
| Embryonic development study, rats | Rat [Female] | Oral(gavage) | 0,5, 17 + 60 |
| Embryonic development study, rabbits | Rabbit [Female] | Oral(gavage) | 0, 5, 30 + 70 |

In the following the methods used and the results gained will be described in detail for each above mentioned toxicity study:

### Example 3a: Embryonic development study in rats:

Pregnant rats received 0, 5, 17, and 60 mg/kg of the compound N-{[17-(5-Fluoropyridin-3-yl)estra-1,3,5(10),16-tetraen-3-yl]carbonyl}-N-methyl-β-alanine once daily oral by gavage from Day 6 to Day 17 of gestation. The control received the vehicle in the same manner. All F0-females were sacrificed on Day 21 of gestation and the following parameters were recorded after removal and examination of the ovaries and the uteri: Number of corpora lutea, number and state of implantation sites (i.e, live foetus, early and late interauterine deaths, dead fetuses). Early interauterine deaths were classified as those which showed decidual or placental tissue only. Late intrauterine deaths showed embryonic or foetal tissues in addition to placental tissue. Dead fetuses were classified as those which appeared to have died shortly before necropsy. Live foetuses were euthanised by a subcutaneous injection of sodium pentobarbitone. Individual foetal and placental weights were recorded and foetuses were examined for external abnormalities and sexed by external observation. Afterwards, approximately one half of the foetuses in each litter were examined for visceral abnormalities by microdissection. They were then eviscerated and the carcasses processed and Alizarin Red S stained for skeletal examination. The remaining foetuses were fixed in Bouin's fluid and examined for visceral abnormalities by the Wilson sectioning method. Foetal abnormalities were classified as malformations (rare and/or potentially lethal defects) and variations (commonly occurring non-lethal abnormalities).

The most relevant finding noted in foetuses exposed in utero to *N*-{[17-(5-Fluoropyridin-3-yl)estra-1,3,5(10),16-tetraen-3-yl]carbonyl}-*N*-methyl-*β*-alanine was the occurrence of a higher incidence of malformations at the high dose of compound *N*-{[17-(5-Fluoropyridin-3-yl)estra-1,3,5(10),16-tetraen-3-yl]carbonyl}-*N*-methyl-*β*-alanine (60 mg/kg) (12 foetuses from eight dams out of a total of 198 foetuses) compared to the control (1 foetus out of 211). Some of these malformations, which are not included in the historical controls, occurred at a low incidence in the high dose group as one or two fetuses were concerned. These malfromations were related to skeletal formation, to the aortic arch or were confined to one foetus with multiple abnormalities. In addition, at 60 mg/kg a higher incidence of certain fetal visceral and skeletal variations was observed compared to the concurrent or the historical control. Thus, in summary, the NOAEL for maternal toxicity is considered to be 60 mg/kg. The NOAEL for developmental toxicity is regarded as 17 mg/kg since foetal structural findings classified as variations or malformations were induced at the high dose of 60 mg/kg.

### Example 3b: Embryonic development study in rabbits:

Pregnant rabbits were treated with 0, 5, 20 + 70 mg/kg of the compound N-{[17-(5-Fluoropyridin-3-yl)estra-1,3,5(10),16-tetraen-3-yl]carbonyl}-N-methyl-β-alanine once daily oral by gavage from Day 6 to Day 19 of gestation. The control received the vehicle in the same manner. All F0-females were sacrificed on Day 29 of gestation and the following parameters were recorded after removal and examination of the ovaries and the uteri: Number of corpora lutea, number and state of implantation sites (i.e, live foetus, early and late interauterine deaths, dead fetuses). Early interauterine deaths were classified as those which showed decidual or placental tissue only. Late intrauterine deaths showed embryonic or foetal tissues in addition to placental tissue. Dead fetuses were classified as those which appeared to have died shortly before necropsy. Live foetuses were euthanised by an intraperitoneal injection of sodium pentobarbitone. Individual foetal and placental weights were recorded and foetuses were examined for external abnormalities and sexed by external observation. All fetuses in each litter were examined for visceral abnormalities by microdissection, eviscerated and the carcasses processed and Alizarin Red S stained for skeletal examination. The intact heads of approximately one half of the fetuses in each litter were removed after the fetuses were killed and were processed in Bouin's fluid. The heads were then examined by the Wilson sectioning method. Foetal abnormalities were classified as malformations (rare and/or potentially lethal defects) and variations (commonly occurring non-lethal abnormalities). The most relevant finding noted in rabbit foetuses which were exposed in utero to the N-{[17-(5-Fluoropyridin-3-yl)estra-1,3,5(10),16-tetraen-3-yl]carbonyl}-N-methyl-β-alanine was the occurrence of foetal skeletal findings classified as variations occurred at the mid dose of 20 mg/kg and higher. These variations were related to sternebra. They included sternebra with an additional ossification site recorded at a higher incidence compared with concurrent and historical control, as well as the occurrence of sternebra with increased ossification which was observed in all groups that received the compound N-{[17-(5-Fluoropyridin-3-yl)estra-1,3,5(10),16-tetraen-3-yl]carbonyl}-N-methyl-β-alanine but at a very low incidence at 5 mg/kg. This finding was not present in the background data. In addition, a slightly higher incidence of a discoloured thymus was observed in fetuses at 70 mg/kg compared to the historical control data. Thus, in summary, the NOAEL for maternal toxicity is considered to be 70 mg/kg. The NOAEL for developmental toxicity is regarded as 5 mg/kg due to the occurrence of foetal findings classified as variations at the dose of 20 mg/kg and higher.

## Claims

1. An oral dosage form comprising up to 120 mg *N*-{[17-(5-Fluoropyridin-3-yl)estra-1,3,5(10), 16-tetraen-3-yl]carbonyl}-*N*-methyl-*β*-alanine.

2. An oral dosage form according to claim 1 whereby the dosage is 120 mg.

3.4. An oral dosage form according to claim 1 whereby the dosage is 60 mg.

4. An oral dosage form according to claim 1 whereby the dosage is 30 mg.

5. An oral dosage form according to claim 1 whereby the dosage is 15 mg.

6. An oral dosage form according to claim 1 whereby the dosage is 3 mg.

7. An oral dosage form according to claim 1 whereby the dosage is 1 mg.

8. An oral dosage form according to any of claims 1 to 7, wherin said dosage form is a tablet.

9. A dosage form according to claim 8, wherin said tablet is film-coated.

10. An oral dosage form according to any of claims 1 to 7, wherin said dosage form is liquid.

11. The dosage form according to any of claims 1 to 10 for use as a medicament.

12. The dosage form according to any of claims 1 to 10 for the prevention, treatment or alleviation of endometriosis, polycystic ovary syndrome and atopic dermatitis.

13. A method for preventing, treating or alleviating endometriosis, polycystic ovary syndrome and atopic dermatitis, said method comprising administering a dosage form as defined in any of claims 1 to 10 to a woman in need thereof.
